# EUROPÄISCHE PATENTSCHRIFT

(11) **EP 1 909 100 B1**
(45) Veröffentlichungstag und Bekanntmachung des Hinweises auf die Patenterteilung: **07.01.2015**
(21) Anmeldenummer: 07018578.0
(22) Anmeldetag: 21.09.2007
(51) Int. Cl.: G01N 33/30

(54) **Prüfverfahren zum Ermitteln einer Ölseparationsneigung von Schmierfetten und Prüfvorrichtung zu dessen Durchführung**
Test method for determining the oil separation proclivity of lubricating grease and test device for performing the same
Procédé de vérification destiné à la détermination d'une tendance à la séparation de l'huile de lubrifiants et dispositif de vérification destiné à sa réalisation

(30) Priorität: 02.10.2006 DE 102006047024
(43) Veröffentlichungstag der Anmeldung: 09.04.2008
(73) Patentinhaber: SKF Lubrication Systems Germany AG, 12277 Berlin (DE)
(72) Erfinder: Stockhammer, Raimund, 12205 Berlin (DE); Kömmler, Andreas, 12305 Berlin (DE)
(74) Vertreter: Grünecker, Kinkeldey, Stockmair & Schwanhäusser

(56) Entgegenhaltungen:
- DE-A1- 2 018 265
- DE-U1- 9 416 284
- US-A- 1 665 933
- US-A- 3 946 593
- US-A- 5 679 883

## Beschreibung

Die Erfindung betrifft ein Prüfverfahren zum Ermitteln einer Ölseparationsneigung von Schmierfetten unter einer Druckbelastung, bei dem ein mit Schmierfett gefülltes Probevolumen über einen vorbestimmten Prüfzeitraum mit einem vorbestimmten Prüfdruck beaufschlagt, das Schmierfett des Probevolumens mit einem Ölabsorptions- oder Ölausscheideorgan in Kontakt gebracht und die Änderung des Probevolumens aufgrund einer Absorption oder Ausscheidung von aus dem Schmierfett separiertem Öl durch das Ölabsorptions- oder Ölausscheideorgan am Ende des Prüfzeitraums ermittelt wird.

Die Erfindung betrifft ferner eine Prüfvorrichtung zur Ermittlung einer Ölseparationsneigung von Schmierfetten unter einer Druckbelastung, mit einem Probevolumen zur Aufnahme einer Schmierfettprobe, mit einem das Probevolumen begrenzenden Druckkolben, durch den das Probevolumen mit einem vorbestimmten Prüfdruck beaufschlagbar ausgebildet ist, mit einem mit dem Probevolumen ölabsorbierend oder ölausscheidend gekoppelten Ölabsorptions- oder Ölausscheideorgan und mit einem Messmittel, durch das eine für eine Änderung des Probevolumens repräsentative Größe messbar ist.

In Zentralschmieranlagen wird Schmierfett von einem zentralen Reservoir über ein Leitungssystem zu einer Vielzahl von Schmierstellen geleitet. Die Zentralschmieranlagen umfassen Schmierstoffverteiler, beispielsweise Progressivverteiler, die eine vorbestimmte Schmierfettmenge auf die unterschiedlichen Schmierstellen verteilen. Schmierfette bestehen im Wesentlichen aus einer festen Phase, dem Eindicker, und einer flüssigen Phase, dem Öl. Beide Phasen werden während des Schmierstoffherstellungsprozesses mehr oder weniger gut und haltbar miteinander vermengt bzw. verbunden.

Bei Zentralschmieranlagen kann es zu Funktionsstörungen kommen, wenn es zwischen einzelnen Schmierintervallen zum sogenannten Ausbluten des Schmierfetts durch den Restdruck in der Anlage kommt. Beim Ausbluten kommt es zur mehr oder weniger starken Trennung von Öl und Eindicker. Da das Öl aufgrund seiner Konsistenz entweichen kann, beispielsweise durch einen Dichtspalt, bleibt der Eindicker zurück und löst schlimmstenfalls eine Funktionsstörung der Zentralschmieranlage aus, z.B. durch ein Blockieren eines Schmierstoffverteilers. Die Folge sind Stillstandzeiten der zu schmierenden Maschine oder Anlage mit den entsprechenden Folgeerscheinungen, wie Produktionsausfall, ungeplante Wartungsarbeiten usw. Unter Umständen müssen Schmierstoffverteiler an möglicherweise unzugänglichen Stellen ausgewechselt werden.

Um den Ausfall einer Zentralschmieranlage aufgrund von einer Ölseparation des Schmierfettes zu vermeiden, werden Schmierfette vor ihrem Einsatz in einer Zentralschmieranlage auf ihre Ölseparationsneigung getestet.

In der DE 94 16 284 U1 ist eine Prüfvorrichtung beschrieben, mit der Schmierfette auf ihre Ölseparationsneigung getestet werden können. Mit der in der DE 94 16 284 U1 beschriebenen Prüfvorrichtung kann ermittelt werden, ob ein Schmierfett für den Einsatz in Zentralschmieranlagen geeignet ist oder nicht.

Zwar liefert das Prüfungsverfahren mit der Vorrichtung der DE 94 16 284 U1 eine Aussage, ob ein Schmiermittel einen für den Einsatz in einer Zentralschmieranlage vorbestimmten Grenzwert der Ölseparationsneigung übersteigt und somit generell für den Einsatz in der Zentralschmleranlage geeignet ist oder nicht. Jedoch sind nicht alle Zentralschmieranlagen vergleichbar und das Verfahren nach der DE 94 16 281 U1 liefert keine spezifische Aussage darüber, ob ein Schmierfett z.B. für eine Zentralschmieranlage mit besonders kurzen Schmierintervallen nicht doch geeignet ist.. Um eine möglichst große Anzahl an Schmierfetten trotz Separationsneigung fördern zu können, besteht der Wunsch herauszufinden, wann Schmierfette mit höherer Ölseparationsneigung in Zentralschmieranlagen eingesetzt werden können.

Folglich liegt der Erfindung die Aufgabe zugrunde, ein Prüfverfahren und eine Prüfvorrichtung zur Durchführung des Prüfverfahrens bereitzustellen, mit dem die Ölseparationsneigung eines Schmierfettes genauer als bisher bestimmt werden kann.

Diese Aufgabe wird für das erfindungsgemäße Prüfverfahren dadurch gelöst, dass der zeitliche Verlauf der für die Ölseparation repräsentativen Änderung des Probevolumens über den Prüfzeitraums erfasst wird.

Für die erfindungsgemäße Prüfvorrichtung wird die Aufgabe durch eine mit dem Messmittel signalübertragend verbundenen Überwachungseinrichtung gelöst, die eingerichtet ist, um einen zeitlichen Verlauf der Änderung des Probevolumens zu erfassen.

Die erfindungsgemäße Lösung hat den Vorteil, dass anhand des zeitlicher Verlauf der Änderung des Probevolumens, das für die Ölseparation repräsentativ ist, eine maximal zulässige Verweilzeit des Schmierfetts in einer Zentralschmiereinrichtung bestimmt werden kann. Aus dieser maximal zulässigen Verweilzeit kann eine Vorgabe für die Steuerzeiten, wie z.B. Förderzeit und Pausenzeit, einer Zentralschmieranlage abgeleitet werden. So können auch Schmierstoffe mit einer erhöhten Ölseparationsneigung eingesetzt werden, wenn eine maximal zulässige Verweilzeit in dem Schmiersystem nicht überschritten wird.

Das erfindungsgemäße Prüfverfahren sowie die erfindungsgemäße Prüfvorrichtung können durch verschiedene, voneinander unabhängige, jeweils für sich vorteilhafte Ausgestaltungen weiterentwickelt werden. Auf diese Ausgestaltungen und die mit den Ausgestaltungen jeweils verbundenen Vorteile wird im Folgenden kurz eingegangen.

So kann bei dem erfindungsgemäßen Prüfverfahren die Volumenänderung des Probevolumens durch das Messen einer für das Probevolumen repräsentativen Größe ermittelt werden. Dies hat den Vorteil, dass sich das erfindungsgemäße Prüfverfahren bei gleichbleibender Genauigkeit einfacher durchführen lässt, weil eine direkte Volumenmessung nur schwer möglich ist. Als repräsentative Größe kann beispielsweise die Relativbewegung des Druckkolbens der Prüfvorrichtung überwacht werden, da der Druckkolben der Volumenänderung des Probevolumens folgt.

Um einen zur Steuerung einer Zentralschmieranlage verwendbaren Parameter zu erhalten, kann aus dem zeitlichen Verlauf ein Grenzzeitpunkt, bei dem die Ölseparation einen vorbestimmten Grenzwert übersteigt, bestimmt werden. Ferner können der zeitlicher Verlauf der Änderung des Probevolumens sowie der Grenzzeitpunkt gespeichert werden, um einen späteren Zugriff zu ermöglichen.

Um die Ölseparationsneigung eines Fettes in Abhängigkeit vom Druck und/oder der Temperatur zu ermitteln, können der Prüfdruck und/oder die Temperatur des Schmierfettes im Probevolumen innerhalb des Prüfzeitraumes im Wesentlichen konstant gehalten werden.

Für ein Prüfverfahren zum Ermitteln der Ölseparationsneigung eines Schmierfettes für unterschiedliche Temperaturen und/oder Prüfdrücke, kann das oben genannte Verfahren mit seinen verschiedenen Ausführungsformen mit veränderter Temperatur und/oder verändertem Prüfdruck wiederholt werden. So kann eine Kurvenschar für die Ölseparationsneigung eines Schmierfettes über die Zeit und unterschiedliche Grenzzeitpunkte ermittelt werden, wobei jede Kurve und jeder Grenzzeitpunkt eine bestimmte Temperatur und einen bestimmten Prüfdruck repräsentiert. Mit diesen spezifischen Grenzzeitpunkten kann eine Zentralschmieranlage gesteuert werden.

In einer vorteilhaften Ausgestaltung der erfindungsgemäßen Prüfvorrichtung kann die Überwachungseinrichtung einen Grenzzeitpunkt, bei dem die Ölseparation einen vorbestimmten Grenzwert übersteigt, erfassend ausgebildet sein. Dies hat den Vorteil, dass der Grenzzeitpunkt als Steuerungsparameter einer Zentralschmieranlage genutzt werden kann.

Weiterhin kann die Prüfvorrichtung eine Temperierungseinrichtung zur Einstellung einer vorbestimmten Temperatur des Probevolumens aufweisen. Dies hat den Vorteil, dass die Prüfung der Schmierfettprobe mit unterschiedlichen, vorbestimmbaren Temperaturen durchgeführt werden kann und so eine Aussage über die Temperaturabhängigkeit der Ölseparationsneigung getroffen werden kann. Die Temperierungseinrichtung kann entweder nur das Prüfvolumen mit der Schmierfettprobe oder beispielsweise den gesamten Raum, in dem die Prüfvorrichtung angeordnet ist, temperieren.

Ferner kann die Überwachungseinrichtung ein Speichermittel und/oder ein Anzeigemittel aufweisen, durch die der zeitliche Verlauf der Änderung des Probevolumens und der Grenzzeitpunkt speicherbar und/oder anzeigbar sind.

Um während einer Prüfung den nötigen Prüfdruck zu erzeugen, kann die Prüfvorrichtung ein Kraftelement aufweisen, durch das eine im Wesentlichen konstante Kraft auf den Druckkolben ausübbar ist. Ein solches Kraftelement kann beispielsweise ein Pneumatikzylinder, ein Gewicht oder eine Feder sein.

In einer vorteilhaften Weiterbildung kann die Überwachungseinrichtung aus den Messwerten des Messmittels, d.h. den Positionen des Druckkolbens, eine Messkurve erzeugen. Dies hat den Vorteil, dass aus der Messkurve zu jedem Zeitpunkt eine bestimmte Größe an Ölseparation abgelesen werden kann, selbst wenn das Messmittel z.B. nur jede fünf Minuten einen Messwert ausgibt. Die Messkurve kann z.B. durch Interpolation erzeugt und beispielsweise geglättet werden. So kann der Grenzzeitpunkt genauer bestimmt werden. Ferner kann die Überwachungseinrichtung eine Kurvenschar aus mehreren Messkurven erzeugen, die jeweils bei unterschiedlichen Prüftemperaturen und/oder Prüfdrücken für ein Schmierfett ermittelt wurden. Aus einer solchen Kurvenschar eines Schmierfettes können Steuerparameter für eine Zentralschmieranlage bestimmt werden, wie z.B. die Häufigkeit von Schmierintervallen in Abhängigkeit von einem Restdruckniveau, einer Verweilzeit des Schmierfetts und einer Schmierfetttemperatur.

Die Erfindung betrifft neben dem oben beschriebenen Prüfverfahren und der Prüfvorrichtung auch ein Verfahren zur Steuerung einer Zentralschmieranlage, bei dem in Schmierintervallen eine bestimmte Schmierstoffmenge durch Schmierstoffleitungen zu mehreren Schmierstellen gefördert wird. Um Schmierfette mit einer Ölseparationsneigung einsetzen zu können, ohne dass es zu einem Ausfall der Zentralschmieranlage durch Ölseparation kommt, ist erfindungsgemäß vorgesehen, dass der zeitliche Abstand zwischen den Schmierintervallen in Abhängigkeit von einem Grenzzeitpunkt, bei dem der gemäß einem erfindungsgemäßen Prüfverfahren ermittelte zeitliche Verlauf der Ölseparation des Schmierfettes einen vorbestimmten Grenzwert übersteigt, gesteuert wird.

Um eine Ölseparation des Schmierfettes in der Zentralschmieranlage noch sicherer ausschließen zu können, kann der zeitliche Abstand der Schmierintervalle in Abhängigkeit von der Schmierfetttemperatur und/oder dem Druck in der Zentralschmieranlage gesteuert werden.

Im Folgenden wird die Erfindung beispielhaft mit Bezug auf die beigefügten Zeichnungen erläutert. Die unterschiedlichen Merkmal können dabei unabhängig voneinander kombiniert werden, wie dies oben bei den einzelnen vorteilhaften Ausgestaltungen bereits dargelegt wurde.

Es zeigen:
- Fig. 1: eine schematische Darstellung einer erfindungsgemäßen Prüfvorrichtung in einer ersten Position;
- Fig. 2: die erfindungsgemäße Prüfvorrichtung aus Fig. 1 in einer zweiten Position;
- Fig. 3: die Prüfvorrichtung aus Fig. 1 und 2 in einer dritten Position;
- Fig. 4: ein Diagramm der Ölseparation eines Schmierfettes, ermittelt mit der Prüfvorrichtung der Fig. 1 bis 3;
- Fig. 5: eine schematische Darstellung einer erfindungsgemäßen Zentralschmieranlage.

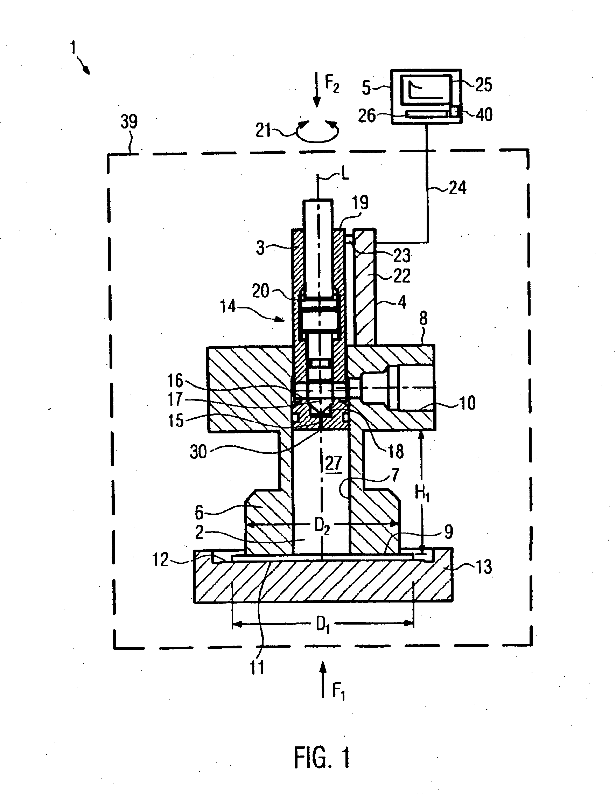

Zunächst wird der allgemeine Aufbau einer erfindungsgemäßen Prüfvorrichtung 1 mit Bezug auf Fig. 1 und der darin dargestellten beispielhaften Ausführungsform beschrieben.

Die Prüfvorrichtung 1 umfasst ein Prüfvolumen 2, einen das Prüfvolumen 2 begrenzenden Druckkolben 3, ein Messmittel 4 sowie eine mit dem Messmittel 4 signalübertragend verbundene Überwachungseinheit 5.

Das zylindrische Prüfvolumen 2 ist in einem Prüfzylinder 6 mit einer Zylinderbohrung 7 ausgebildet. Am oberen Ende ist das Prüfvolumen 2, das eine Prüfkammer darstellt, von dem in der Zylinderbohrung 7 angeordneten Druckkolben 3 begrenzt. Der Prüfzylinder 6 ist bei der Ausführungsform in Fig. 1 rotationssymmetrisch um eine Längsachse L ausgebildet und weist im Wesentlichen quer zur Längsachse L verlaufende, ebene Stirnflächen 8, 9 auf. Quer zur Längsrichtung L ist in dem Prüfzylinder 6 eine Entsorgungsbohrung 10 ausgebildet, die in die Zylinderbohrung 7 mündet. Bei der Ausführungsform in Fig. 1 verjüngt sich die Entsorgungsbohrung 10 von der Außenumfangsfläche des Prüfzylinders 6 in Richtung der Zylinderbohrung 7 stufenweise.

Das Prüfvolumen 2 ist an dem vom Druckkolben 3 gegenüberliegenden Ende von einem Ölabsorptions- oder Ölausscheideorgan 11 begrenzt. Das Ölabsorptions- oder Ölausscheideorgan 11 ist in einer Tasche 12 einer Druckscheibe 13 aufgenommen. Das scheibenförmige Ölabsorptions- oder Ölausscheideorgan 11 weist einen größeren Außendurchmesser D1 auf als der Außendurchmesser D2 der unteren Stirnfläche 9 des Prüfzylinders 6, so dass das Ölabsorptions- oder Ölausscheideorgan 11 seitlich über den Prüfzylinder 6 hinausragt. Das Ölabsorptions- oder Ölausscheideorgan 11 ist in der Längsrichtung L zwischen dem Prüfzylinder 6 und der Druckscheibe 13 angeordnet. Bei der Ausführungsform in Fig. 1 ist die Druckscheibe 13 mit einer in der Längsrichtung L wirkenden Kraft F1 gegen den Prüfzylinder 6 gedrückt, so dass das Ölabsorptions- oder Ölausscheideorgan 11 zwischen der Druckscheibe 13 und dem Prüfzylinder 6 eingeklemmt ist. Die Druckscheibe 13 kann mit Befestigungsmitteln (nicht dargestellt), wie z.B. Schrauben, mit dem Prüfzylinder 6 verbunden sein.

Das Öfabsorptions- oder Ölausscheideorgan 11 ist bei der Ausführungsform in Fig. 1 als ein Rundfilterelement aus einem saugfähigen Material, wie beispielsweise einem Filterpapier, hergestellt. Alternativ kann das Ölabsorptions- oder Ölausscheideorgan 11 aus einem beliebigen saugfähigen Material hergestellt sein, das separiertes Öl absorbieren oder aus dem Prüfvolumen 2 herausleiten kann. Ebenfalls alternativ kann das Ölausscheideorgan 11 auch als ein Spalt, durch den das Öl entweichen kann und/oder der das Schmierfett zurückhält, ausgebildet sein.

Der das Prüfvolumen 2 nach oben begrenzende Druckkolben 3 ist im Wesentlichen kreiszylindrisch ausgebildet und weist eine an das Prüfvolumen 2 grenzende Kolbenfläche 30, eine Düsenbohrung 15, eine Querbohrung 16 und eine Ventileinheit 14 auf. Die auf der Längsachse L verlaufende Düsenbohrung 15 mündet in eine quer zur Längsrichtung L verlaufende Querbohrung 16. Die Ventileinheit 14 ist in diesem Mündungsbereich zwischen der Querbohrung 16 und der Düsenbohrung 15 ausgebildet und verschließt in der Position in Fig. 1 die Verbindung zwischen der Düsenbohrung 15 und der Querbohrung 16. Die Ventileinheit 14 umfasst einen Ventilstößel 17 und einen Ventilsitz 18. Der Ventilstößel 17 ist in einer in Längsrichtung L verlaufenden Ventilbohrung 19 im Druckkolben angeordnet und über ein Gewinde 20 mit dem Druckkolben 3 verbunden. Mittels einer Drehbewegung 21 kann der Ventilstößel 17 mittels dem Gewinde 20 relativ zum Druckkolben 3 entlang der Längsachse L vor und zurück, d.h. in den Ventilsitz 18 hinein und heraus, bewegt werden. Somit kann durch die Ventileinheit 14 die Verbindung zwischen der Düsenbohrung 15 und der Querbohrung 16 geöffnet bzw. geschlossen werden. Der Druckkolben 3 ist so zum Prüfzylinder 6 ausgerichtet, dass die Querbohrung 16 in der in Fig. 1 dargestellten ersten Hubposition mit der Entsorgungsbohrung 10 fluchtet. In der ersten Hubposition weist die im Wesentliche ebene Kolbenfläche 30 einen Abstand H1 zum Ölabsorptions- oder Ölausscheideorgan 11 auf.

Der Druckkolben 3 ist entlang der Längsrichtung L in der Zylinderbohrung 7 linear beweglich angeordnet. Der Außendurchmesser des Druckkolbens 3 ist im Wesentlichen gleich mit dem Innendurchmesser der Zylinderbohrung 7 ausgebildet, so dass im Wesentlichen kein Schmierfett aus dem Probevolumen 2 entweichen kann und der Prüfdruck konstant gehalten werden kann. Die Zylinderbohrung 7 dient als Führungsfläche für den Druckkolben 3.

Das Messmittel 4 der Prüfvorrichtung 1 ist bei der Ausführungsform in Fig. 1 auf der oberen Stirnfläche 8 des Prüfzylinders 6 angeordnet. Das Messmittel 4 umfasst einen statischen Teil 22 und einen zum statischen Teil 22 entlang der Längsrichtung L beweglichen Teil 23. Der statische Teil 22 ist mit dem Prüfzylinder 6 und der bewegliche Teil 23 mit dem Druckkolben 3 jeweils bewegungsübertragend verbunden. Das Messmittel 4 ist als ein Längenmessmittel, z.B. ein handelsübliches inkrementales oder analoges Wegmesssystem, ausgebildet, und erfasst eine Relativbewegung des Druckkolbens 3 zum Prüfzylinder 6.

Das Messmittel 4 ist über eine Verbindungsleitung 24 signalübertragend mit der Überwachungseinheit 5 verbunden. Die Überwachungseinheit 5 umfasst eine Steuerungseinheit 40 ein Anzeigemittel 25 und ein Speichermittel 26. Die Steuerungseinheit 40 verarbeitet die Signale des Messmittels 4 und ist mit dem Anzeigemittel 25 und dem Speichermittel 26 verbunden. Das Anzeigemittel 25 stellt das Messsignal des Messmittels 4 grafisch dar und ist beispielsweise ein Monitor. Das Speichermittel 26 speichert die Messsignale des Messmittels 4 bzw. die graphische Darstellung für einen späteren Zugriff oder eine spätere Verarbeitung ab. Das Speichermittel 26 ist beispielsweise ein Festplattenspeicher. Die Überwachungseinheit 5 ist beispielsweise ein PC, an den das Messmittel 4 über einen Schnittstellenwandler angeschlossen ist. Die Messsignale können im PC z.B. in eine Tabellenkalkulationssoftware geschrieben und mittels dieser Software graphisch dargestellt werden.

Die Prüfvorrichtung 1 umfasst weiterhin eine Temperierungseinrichtung 39, welche die Temperatur innerhalb des Probevolumens 2 auf einer vorbestimmten, veränderbaren Temperatur hält. In Fig. 1 temperiert die Temperierungseinrichtung 39 die gesamte Prüfvorrichtung 1. Alternativ kann auch nur das Probevolumen 2 temperiert werden.

Im Betrieb der Prüfvorrichtung 1 wird zur Durchführung des erfindungsgemäßen Prüfverfahrens das Prüfvolumen 2, wie in Fig. 1 dargestellt, mit einem zu prüfenden Schmierfett 27 befüllt. Dabei ist der Druckkolben 3 in eine, in Fig. 1 dargestellte, Startposition gefahren. Das Prüfvolumen 2 kann bei geschlossene Ventileinheit 14 und bei der in Fig. 1 dargestellten Kolbenposition (H₁) über den nach unten offenen und für die Befüllung nach vorne geschwenkten Prüfzylinder 6 befüllt werden. Nach der Befüllung wir der Zylinder 6 wieder in die in Fig. 1 dargestellte Position geschwenkt und mit dem Ölausscheidungsorgan 11 in der Druckscheibe 13 verschlossen.

Nun wird der Prüfvorgang gestartet, indem eine im Wesentlichen konstante Kraft F₂ auf den Druckkolben 3 ausgeübt wird, z.B. durch einen Pneumatikzylinder, ein Gewicht oder eine Feder. So wird das Prüfvolumen 2 mit dem Schmierfett 27 mit einem im Wesentlichen konstanten Prüfdruck beaufschlagt. Über einen vorbestimmten Prüfzeitraum, beispielsweise 24 Stunden, wird der Prüfdruck in dem Prüfvolumen 2 im Wesentlichen konstant gehalten. Durch die Temperierungseinrichtung 39 wird auch die Temperatur der Schmierfettprobe 27 im Prüfvolumen 2 konstant gehalten.

Schmierfette bestehen im Wesentlichen aus einer festen Phase, dem Eindicker, und einer flüssigen Phase, dem Öl. Beide Phasen werden während des Schmierstoffherstellprozesses mehr oder weniger gut und haltbar miteinander vermengt bzw. verbunden. Unter Druckeinfluss kann es mehr oder weniger stark zur Trennung der beiden Phasen kommen. Diese Trennung wird als Ölseparation bezeichnet. Die Ölseparation der Schmierfettprobe 27 und ein Zeitpunkt T, bei dem die Ölseparation einen vorbestimmten Grenzwert H_{G} übersteigt, wird durch das erfindungsgemäße Prüfverfahren mit der Prüfvorrichtung 1 über den Prüfzeitraumes ermittelt. Eine Ölseparation oberhalb des Grenzwertes H_{G} kann für den Betrieb einer Zentralschmieranlage kritisch sein.

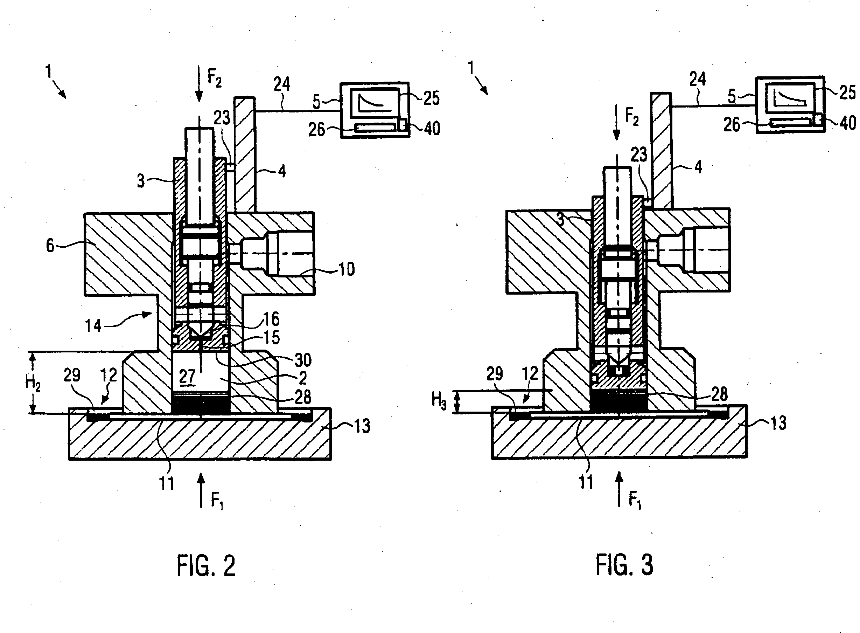

Fig. 2 zeigt die erfindungsgemäße Prüfvorrichtung 1 am Ende des Prüfzeitraums. Gegenüber Fig. 1 hat sich im Prüfvolumen 2 in Fig. 2 eine Schichtung aus noch weichem Schmierfett 27 und bereits ausgehärtetem Schmierfett 28 ausgebildet. Weiterhin hat sich in der Tasche 12 separiertes Öl 29 um das Ölabsorptions- oder Ölausscheideorgan 11 gesammelt. Der Druckkolben 3 befindet sich in Fig. 2 in einer Hubposition, in der die Kolbenfläche 30 einen Abstand H₂ zum Ölabsorptions- oder Ölausscheideorgan 11 aufweist. Der Abstand H₂ am Ende des Prüfzeitraums ist kleiner als der Abstand H₁, den die Kolbenfläche 30 am Anfang des Prützeitraums in Fig. 1 eingenommen hatte.

Innerhalb des Prüfzeitraums hat das Messmittel 4 die Position des Druckkolbens 3 kontinuierlich erfasst und als Messsignal an die Überwachungseinheit 5 übermittelt. Die Überwachungseinheit 5 hat die Messsignale des Messmittels 4 zu einer Messkurve verarbeitet.

Fig. 3 zeigt die erfindungsgemäße Prüfvorrichtung 1 ebenfalls nach Beendigung des Prüfzeitraums, allerdings in einer Position, in der das in Fig. 2 noch vorhandene weiche bzw. fließfähige Schmierfett 27 aus der Prüfvolumen 2 entfernt worden ist. Hierzu wurde in der Position in Fig. 2 das Sitzventil 14 geöffnet, so dass die Düsenbohrung 15 mit der Entsorgungsbohrung 10 verbunden ist, weil der Druckkolben 3 am Außendurchmesser oberhalb der Querbohrung 16 verjüngt ist. Da die Kraft F₂ weiterhin auf den Druckkolben 3 wirkt und somit das Schmierfett 27 in der Prüfvolumen 2 weiter druckbeaufschlagt ist, entweicht das Schmierfett 27 aus der Entsorgungsbohrung 10 und der Druckkolben 3 bewegt sich in die in Fig. 3 dargestellte Position, in der nur noch ausgehärtetes, nicht mehr fließfähiges Schmierfett im Probevolumen 2 vorhanden ist. In Fig. 3 weist die Kolbenfläche 30 einen Abstand H₃ zum Ölabsorptions- oder Ölausscheideorgan 11 auf. Der Abstand H₃ ist kleiner als die Abstände H₂ und H₁. Bei der in Fig. 3 dargestellten Position der Prüfvorrichtung 1 befindet sich nur noch ausgehärtetes, nicht mehr fließfähiges Schmierfett in dem Prüfvolumen 2 und die Kolbenfläche 30 liegt auf ausgehärteten Schmierfettpfropfen 28 auf. Somit entspricht die Höhe H₃ der Höhe des ausgehärteten Schmierfettpfropfens 28 in dem Prüfvolumen 2. Anschließend werden die Aushärthöhe H₃ und die Höhe H₂, welche die Ölseparation repräsentiert, zur Höhe H₁, die das Gesamtvolumen repräsentiert, ins Verhältnis gesetzt. So wird der vermutliche Eindickeranteil des Schmierfettes kalkuliert.

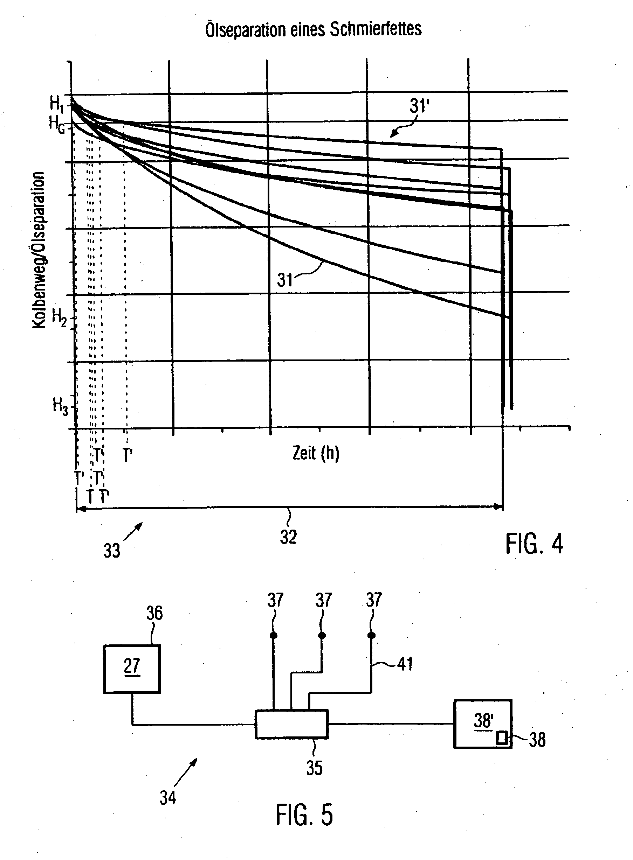

Fig. 4 zeigt zwei Messkurven 31, 31', die durch die Überwachungseinheit 5 während zwei Prüfvorgängen aufgezeichnet worden sind. Die Messkurven 31, 31' sind in dem Diagramm in Fig. 4 in einem kartesischen Koordinatensystem mit dem Kolbenweg bzw. der Ölseparation als Ordinate und der Zeit als Abszisse aufgezeigt. Die Messkurve 31 zeigt den Weg des Kolbens innerhalb des Prüfzeitraums 32 Die in Fig. 4 dargestellten Messkurven 31, 31' zeigen jeweils die mit der Zeit zunehmende Ölseparation als Funktion des zeitlichen Verlaufs des Kolbenweges von zwei unterschiedlichen Schmierstoffen. Zusätzlich können beispielsweise weitere Prüfungen mit unterschiedlichen Temperaturen und/oder Prüfdrücken für gleiche Schmierstoffe durchgeführt werden.

In Figur 4 ist ein Grenzwert H_{G} eingezeichnet, der eine empirisch ermittelte, gerade noch zulässige Ölseparation repräsentiert. Die Messkurve 31' eines Schmierstoffs A liegt im dargestellten Zeitraum stets unterhalb des Grenzwertes H_{G} Dieser Schmierstoff kann somit als unkritisch bezüglich der Ölseparation eingestuft werden und wäre auch durch die bekannten Prüfverfahren zur Ermittlung der Ölseparationsneigung als zulässig eingestuft worden. Die Messkurve 31 eines anderen Schmierstoffs B überschreitet den Grenzwert H_{G} zu einem Zeitpunkt T. Durch die Bestimmung des zeitlichen Verlaufs der Ölseparation und des Zeitpunkts T mit dem erfindungsgemäßen Prüfverfahren ergibt sich, dass der Schmierstoff B bis zum Erreichen des Zeitpunkts T in einer Zentralschmieranlage gefördert werden kann, ohne dass der kritische Grenzwert H_{G} überschritten wird. Nach den bekannten Prüfverfahren wäre der Schmierstoff B als nicht einsatzfähig für eine Zentralschmieranlage eingestuft worden, da er den Grenzwert H_{G} am Ende des Prüfzeitraums 32 überschreitet.

Fig. 5 zeigt eine schematische Darstellung einer erfindungsgemäßen Zentralschmieranlage 34 mit einem Progressivverteiler 35, der Schmierfett 27 aus einem Reservoir 36 mit Hilfe einer Schmiermittelpumpe 38' über Schmierstoffleitungen 41 zu mehreren Schmierstellen 37 leitet. Die Zentralschmieranlage 34 umfasst ferner eine Steuerungseinheit 38, die z.B. in die Pumpe 38' integriert sein kann, die den Progressivverteiler 35 mit Schmierfett versorgt. Das Schmierfett 27 in Fig. 5 ist der oben beschriebene Schmierstoff B mit der Messkurve 31 aus Fig. 4. Um zu verhindern, dass das Schmierfett 27 innerhalb der Zentralschmieranlage 34 zu stark ausblutet, d.h. Öl separiert, werden die Zeitabstände zwischen den einzelnen Schmierintervallen anhand von aus dem Diagramm in Fig. 4 ablesbaren Parametern gesteuert. Die Zeit zwischen den Schmierintervallen ist z.B. so gewählt, dass sie unterhalb des Zeitpunkts T liegen, ab dem die Ölseparation des Schmierfettes den Grenzwert H_{G} überschreitet. Der Zeitpunkt T ist aus dem Diagramm in Fig. 4 ermittelt und als ein Steuerungsparameter In die Steuerungseinheit 38 eingegeben worden. Die Steuerungseinheit 38 erfasst die Temperatur und den Druck innerhalb der Zentralschmieranlage 34, so dass für unterschiedliche Temperaturen und Drücke für die jeweils Maßkurven ermittelt werden können, jeweils ein zugehöriger kritischer Zeitpunkt T eingegeben und verwendet werden kann.

## Patentansprüche

1. Prüfverfahren zum Ermitteln einer Ölseparationsneigung von Schmierfette unter einer Druckbelastung, bei dem ein mit Schmierfett (27) gefülltes Probevolumen (2) über einen vorbestimmten Prüfzeitraum (32) mit einem vorbestimmten Prüfdruck beaufschlagt, das Schmierfett (27) des Probevolumens (2) mit einem Ölabsorptions- oder Ölausscheideorgan (11) in Kontakt gebracht und die Änderung des Probevolumens (2) aufgrund einer Absorption oder Ausscheidung von aus dem Schmierfett (27) separiertem Öl (29) durch das Ölabsorptions- oder Ölausscheideorgan (11) am Ende des Prüfzeitraums (32) ermittelt wird, **dadurch gekennzeichnet, dass** der zeitliche Verlauf der für die Ölseparation repräsentativen Änderung des Probevolumens (2) über den Prüfzeitraum (32) erfasst wird.

2. Verfahren nach Anspruch 1, **dadurch gekennzeichnet, dass** aus dem zeitlichen Verlauf ein Grenzzeitpunkt (T), bei dem die Ölseparation einen vorbestimmten Grenzwert (H_{G}) übersteigt, bestimmt wird.

3. Verfahren nach Anspruch 1 oder 2, **dadurch gekennzeichnet, dass** der zeitliche Verlauf der Änderung des Probevolumens (2) und/oder der Grenzzeitpunkt (T) gespeichert werden.

4. Verfahren nach einem der oben genannten Ansprüche, **dadurch gekennzeichnet, dass** die Volumenänderung des Probevolumens (2) durch das Messen einer für das Probevolumen (2) repräsentativen Größe (H) ermittelt wird.

5. Verfahren nach einem der oben genannten Ansprüche, **dadurch gekennzeichnet, dass** der Prüfdruck und/oder die Temperatur des Schmierfettes (27) innerhalb des Prüfzeitraums (32) im Wesentlichen konstant gehalten werden.

6. Prüfverfahren zum Ermitteln einer temperatur- und/oder druckabhängigen Ölseparationsneigung eines Schmierfettes, bei dem das Verfahren nach einem der oben genannten Ansprüche mit jeweils veränderter Temperatur und/oder verändertem Prüfdruck wiederholt wird.

7. Prüfvorrichtung (1) zur Ermittlung einer Ölseparationsneigung von Schmierfetten unter einer Druckbelastung, mit einem Probevolumen (2) zur Aufnahme einer Schmierfettprobe (27), mit einem das Probevolumen (2) begrenzenden Druckkolben (3), durch den das Probevolumen (2) mit einem vorbestimmten Prüfdruck beaufschlagbar ausgebildet ist, mit einem mit dem Probevolumen (2) ölabsorbierend oder ölausscheidend gekoppelten Ölabsorptions- oder Ölausscheideorgan (11) und mit einem Messmittel (4), durch das eine für eine Änderung des Probevolumens (2) repräsentative Größe (H) messbar ist, **gekennzeichnet durch** eine mit dem Messmittel (4) signalübertragend verbundene Überwachungseinrichtung (5), die eingerichtet ist, um einen zeitlichen Verlauf der Änderung des Probevolumens (2) zu erfassen.

8. Prüfvorrichtung nach Anspruch 7, **dadurch gekennzeichnet, dass** die Überwachungseinrichtung (5) einen Grenzzeitpunkt (T), bei dem die Ölseparation einen vorbestimmten Grenzwert (H_{G}) übersteigt, erfassend ausgebildet ist.

9. Prüfvorrichtung (1) nach Anspruch 7 oder 8, **dadurch gekennzeichnet, dass** die Überwachungseinrichtung (5) ein Speichermittel (26) umfasst, durch das der zeitliche Verlauf der Änderung des Probevolumens (2) und/oder der Grenzzeitpunkt (T) speicherbar ist.

10. Prüfvorrichtung (1) nach einem der oben genannten Ansprüche, **dadurch gekennzeichnet, dass** die Überwachungseinrichtung (5) ein Anzeigemittel (25) aufweist, durch das der zeitliche Verlauf der Änderung des Probevolumens (2) und der Grenzzeitpunkt (T) anzeigbar ist.

11. Prüfvorrichtung (1) nach einem der oben genannten Ansprüche, **dadurch gekennzeichnet, dass** die Prüfvorrichtung (1) eine Temperierungseinrichtung (39) zur Einstellung einer vorbestimmten konstanten Temperatur des Probevolumens (2) aufweist.

12. Prüfvorrichtung nach Anspruch 11, **dadurch gekennzeichnet, dass** die Temperatur veränderbar einstellbar ist.

13. Verfahren zur Steuerung einer Zentralschmieranlage (34), bei dem in Schmierintervallen eine vorbestimmte Schmierfettmenge durch Schmierstoffleitungen (41) zu mehreren Schmierstellen (37) gefördert wird, **dadurch gekennzeichnet, dass** der zeitliche Verlauf der Ölseparation des Schmierfettes gemäß einem Prüfverfahren nach einem der Ansprüche 1-6 ermittelt wird, und dass ein zeitlicher Abstand zwischen den Schmierintervallen in Abhängigkeit von einem Grenzzeitpunkt (T), bei dem dieser zeitliche Verlauf einen vorbestimmten Grenzwert (H_{G}) übersteigt, gesteuert wird.

14. Verfahren nach Anspruch 13, **dadurch gekennzeichnet, dass** der zeitliche Abstand zwischen den Schmierintervallen in Abhängigkeit von der Temperatur und/oder dem Druck in der Zentralschmieranlage (34) gesteuert wird.

## Claims

1. Test method for the determination of an oil separation tendency of lubricating greases under pressure load, wherein a predetermined test pressure is applied to a test volume (2) filled with lubricating grease (27) during a predetermined test period (32), the lubricating grease (27) of the test volume (2) is contacted with an oil absorption or oil separation element (11), and the change of the test volume (2) is detected on the basis of an absorption or separation of oil (29) separated from the lubricating grease (27) by the oil absorption or oil separation element (11) at the end of the test period (32), **characterized in that** the time history of the change of the test volume (2) representative of the oil separation is detected over the test period (32).

2. Method according to claim 1, **characterized in that** a limiting point of time (T) at which the oil separation exceeds a predetermined limiting value (H_{G}) is determined from the time history.

3. Method according to claim 1 or 2, **characterized in that** the time history of the change of the test volume (2) and/or the limiting point of time (T) are stored.

4. Method according to one of the preceding claims, **characterized in that** the volume change of the test volume (2) is detected by measuring a quantity (H) representative of the test volume (2).

5. Method according to one of the preceding claims, **characterized in that** the test pressure and/or the temperature of the lubricating grease (27) are kept essentially constant within the test period (32).

6. Test method for the determination of a temperature- and/or pressure-dependent oil separation tendency of a lubricating grease, in which the method according to one of the preceding claims is repeated, in each case with changed temperature or changed test pressure.

7. Test device (1) for the determination of an oil separation tendency of lubricating greases under pressure load with a test volume (2) for receiving a lubricating grease sample (27), with a pressure piston (3) limiting the test volume (2) by which the test volume (2) is constituted such that a predetermined test pressure can be applied to the same, with an oil absorption or oil separation element (11) coupled to the test volume (2) so as to absorb oil or separate oil, and with a measuring means (4) by which a quantity (H) representative of a change of the test volume (2) can be measured, **characterized by** a monitoring unit (5) connected to the measuring means (4) so as to transmit signals and which is configured to detect a time history of the change of the test volume (2).

8. Test device according to claim 7, **characterized in that** the monitoring unit (5) is designed such that it detects a limiting point of time (T) at which the oil separation exceeds a predetermined limiting value (H_{G}).

9. Test device (1) according to claim 7 or 8, **characterized in that** the monitoring means (5) comprises a storage means (26) by which the time history of the change of the test volume (2) and/or the limiting point of time (T) can be stored.

10. Test device (1) according to one of the preceding claims, **characterized in that** the monitoring means (5) comprises a display means (25) by which the time history of the change of the test volume (2) and the limiting point of time (T) can be displayed.

11. Test device (1) according to one of the preceding claims, **characterized in that** the test device (1) comprises a temperature-control means (39) for adjusting a predetermined constant temperature of the test volume (2).

12. Test device according to claim 11, **characterized in that** the temperature can be variably adjusted.

13. Method for controlling a central lubrication system (34) in which at lubrication intervals, a predetermined amount of lubricating grease is delivered to several lubrication points (37) through lubricant lines (41), **characterized in that** the time history of the oil separation of the lubricating grease is determined in accordance with a test method according to one of claims 1 to 6 and a distance of time between the lubrication intervals is controlled in dependency of a limiting point of time (T) at which the time history exceeds a predetermined limiting value (H_{G}).

14. Method according to claim 13, **characterized in that** the distance of time between the lubrication intervals is controlled in dependency of the temperature and/or the pressure in the central lubrication system (34).

## Revendications

1. Procédé de test destiné à déterminer une tendance à la séparation d'huile dans des graisses de lubrification sous l'effet d'une sollicitation en pression, d'après lequel on soumet un volume-échantillon (2) rempli de graisse de lubrification (27) à une pression de test prédéterminée pendant une durée de test prédéterminée (32), on amène la graisse de lubrification (27) du volume-échantillon (2) en contact avec un organe d'absorption d'huile ou de séparation d'huile (11), et on détermine, à la fin de la durée de test (32), la variation du volume-échantillon (2) en raison d'une absorption ou d'une séparation d'huile (29) séparée de la graisse de lubrification (27) par l'organe d'absorption d'huile ou de séparation d'huile (11), **caractérisé en ce que** l'on relève la loi de variation dans le temps de la variation du volume-échantillon (2) représentative de la séparation d'huile, tout au long de la durée de test (32).

2. Procédé selon la revendication 1, **caractérisé en ce qu'**à partir de la loi de variation dans le temps, on détermine un instant limite (T) pour lequel la séparation d'huile dépasse une valeur limite prédéterminée (H_{G}).

3. Procédé selon la revendication 1 ou la revendication 2, **caractérisé en ce que** l'on mémorise la loi de variation dans le temps de la variation du volume-échantillon (2) et/ou l'instant limite (T).

4. Procédé selon l'une des revendications précitées, **caractérisé en ce que** l'on détermine la variation de volume du volume-échantillon (2) par la mesure d'une grandeur (H) représentative pour le volume-échantillon (2).

5. Procédé selon l'une des revendications précitées, **caractérisé en ce que** l'on maintient sensiblement constantes la pression de test et/ou la température de la graisse de lubrification (27), pendant la durée de test (32).

6. Procédé de test destiné à déterminer une tendance à la séparation d'huile d'une graisse de lubrification, en fonction de la température et/ou de la pression, d'après lequel on répète le procédé selon l'une des revendications précitées, respectivement avec une température modifiée et/ou une pression de test modifiée.

7. Dispositif de test (1) destiné à déterminer une tendance à la séparation d'huile dans des graisses de lubrification sous l'effet d'une sollicitation en pression, comprenant un volume-échantillon (2) destiné à recevoir un échantillon de graisse de lubrification (27), comprenant également un piston de compression (3) délimitant le volume-échantillon (2) et grâce auquel le volume-échantillon (2) peut être soumis à une pression de test prédéterminée, le dispositif comprenant en outre un organe d'absorption d'huile ou de séparation d'huile (11) couplé au volume-échantillon (2) pour l'absorption d'huile ou la séparation d'huile, l'ensemble comprenant également un moyen de mesure (4) par lequel peut être mesurée une grandeur (H) représentative pour une variation du volume-échantillon (2), **caractérisé par** un équipement de surveillance (5), qui est relié au moyen de mesure (4) pour la transmission de signal, et qui est conçu pour relever une loi de variation dans le temps de la variation du volume-échantillon (2).

8. Dispositif de test selon la revendication 7, **caractérisé en ce que** l'équipement de surveillance (5) est réalisé pour relever un instant limite (T) pour lequel la séparation d'huile dépasse une valeur limite prédéterminée (H_{G}).

9. Dispositif de test (1) selon la revendication 7 ou la revendication 8, **caractérisé en ce que** l'équipement de surveillance (5) comprend un moyen de mémoire (26) permettant la mémorisation de la loi de variation dans le temps du volume-échantillon (2) et/ou l'instant limite (T).

10. Dispositif de test (1) selon l'une des revendications précitées, **caractérisé en ce que** l'équipement de surveillance (5) comporte un moyen de visualisation (25) par lequel peuvent être visualisés la loi de variation dans le temps de la variation du volume-échantillon (2) et l'instant limite (T).

11. Dispositif de test (1) selon l'une des revendications précitées, **caractérisé en ce que** le dispositif de test (1) comprend un système permettant de tempérer (39), pour régler une température constante prédéterminée du volume-échantillon (2).

12. Dispositif de test selon la revendication 11, **caractérisé en ce que** la température peut être réglée de manière variable.

13. Procédé de commande d'une installation de graissage ou de lubrification centralisée (34), d'après lequel une quantité prédéterminée de graisse de lubrification est refoulée, selon des intervalles de lubrification, à travers des conduites de lubrification (41) jusqu'à plusieurs points de lubrification (37), **caractérisé en ce que** l'on détermine la loi de variation dans le temps de la séparation de l'huile de la graisse de lubrification conformément à un procédé de test selon l'une des revendications 1 - 6, et **en ce que** l'on commande une durée entre les intervalles de lubrification, en fonction d'un instant limite (T) pour lequel cette loi de variation dans le temps dépasse une valeur limite prédéterminée (H_{G}).

14. Procédé selon la revendication 13, **caractérisé en ce que** l'on commande la durée entre les intervalles de lubrification, en fonction de la température et/ou de la pression dans l'installation de lubrification ou de graissage centralisée (34).
